# EUROPEAN PATENT APPLICATION

(11) **EP 2 233 136 A1**
(43) Date of publication of application: **29.09.2010**
(21) Application number: 09178767.1
(22) Date of filing: 18.08.2006
(51) Int. Cl.: A61K 31/00, A61K 31/122, A61K 31/277, A61K 31/341, A61K 31/357, A61K 31/415, A61K 31/4155, A61K 31/42, A61K 31/422, A61K 31/44, A61K 31/4412, A61K 31/4439, A61K 45/06, A61P 25/24, A61P 25/30

(54) **Use of HPPD Inhibitors in the Treatment of Depression and/or Withdrawal Symptoms Associated with Addictive Drugs**

(62) Divisional of application: 06779171.5
(71) Applicant: Syngenta Limited, Guildford Surrey GU2 7YH (GB)
(72) Inventor: Travis, Kim, Zachary, Macclesfield, Cheshire SK10 4TJ (GB); Posner, John, Bracknell, Berkshire RG42 6YA (GB)
(74) Representative: Harrison Goddard Foote

(57) **Abstract**

The present invention relates to, inter alia, the use of a compound capable of inhibiting 4-hydroxyphenylpyruvate dioxygenase (HPPD) in an animal in the manufacture of a medicament for use in the treatment and/or prevention of depression. The invention also provides for the use of a compound capable of inhibiting HPPD in an animal in the manufacture of a medicament for use in the treatment of the withdrawal symptoms associated with an addictive drug which causes dopamine dependant associative learning disorders in said animal. In a particular embodiment said HPPD inhibitor is selected from the group consisting of the compound depicted as compound 1; 2; and 3.22.

## Description

The present invention relates to, *inter alia,* the use of a 4-hydroxyphenylpyruvate dioxygenase (HPPD) inhibitor in the treatment of depression and/or the treatment of withdrawal symptoms associated with an addictive drug which causes dopamine dependent associative learning disorders. More specifically, the HPPD inhibitor is selected from the group consisting of: compound 1; compound 2; and compound 3.22.

Clinical depression is a state of sadness, melancholia or despair that has advanced to the point of being disruptive to an individual's social functioning and/or activities of daily living. Clinical depression is currently the leading cause of disability in the US as well as other countries, and is expected to become the second leading cause of disability worldwide (after heart disease) by the year 2020, according to the World Health Organization.

The effectiveness of antidepressants such as selective serotonin reuptake inhibitors (SSRI) and tricyclic antidepressants *per se* is modest and there is a therapeutic need for more effective therapies

It would therefore be desirable to provide a pharmaceutical which can alleviate depression. Throughout this specification the term "depression" includes bipolar and unipolar disorders.

All in all the present invention therefore seeks to provide, *inter alia,* a pharmaceutical for use in the treatment of depression which pharmaceutical overcomes and/or ameliorates the problems mentioned herein.

Accordingly the present invention provides, amongst other things, compositions and methods for their use to inhibit 4-hydroxyphenylpyruvate dioxygenase in animals such that an increase in dopamine synthesis and/or turnover is achieved.

According to the present invention there is provided the use of at least one compound capable of inhibiting 4-hydroxyphenylpyruvate dioxygenase (HPPD) in an animal in the manufacture of a medicament for use in the treatment and/or prevention of depression. In a particular embodiment said medicament comprises a compound selected from the group consisting of: compound 1; 2; and 3.22 as disclosed herein. In a particular embodiment said medicament comprises the compound depicted as compound 1.

The present invention still further provides the use as described above wherein said medicament comprises a selective serotonin reuptake inhibitor (SSRI) and/or a tricyclic antidepressant (tricyclic). In a particular embodiment said SSRI is selected from the group consisting of: citalopram, escitalopram, fluoxetine, fluvoxamine, paroxetine, and sertraline or mixtures thereof and said tricyclic is selected from the group consisting of: amitriptyline, clomipramine, dosulepin, dothiepin, doxepin, maprotiline, mianserin, trazodone, trimipramine, amoxapine, imipramine, lofepramine, and nortriptyline or mixtures thereof.

In a particular embodiment said SSRI may be administered separately to the medicament comprising said HPPD inhibitor.

In a particular embodiment said tricyclic may be administered separately to the medicament comprising said HPPD inhibitor.

The present invention still further provides a kit comprising a pharmaceutically effective amount of a compound selected from the group consisting of: compound 1; 2; and 3.22 or a pharmaceutically acceptable salt thereof and a pharmaceutically effective amount of a selective serotonin reuptake inhibitor and/or a tricyclic antidepressant and a means for the delivery thereof to an animal.

The present invention still further provides a pharmaceutical composition comprising as an active ingredient a pharmaceutically effective amount of a compound selected from the group consisting of compound 1; 2; and 3.22 or a pharmaceutically acceptable salt thereof and a pharmaceutically effective amount of a selective serotonin reuptake inhibitor and/or a tricyclic antidepressant together with a pharmaceutically acceptable diluent or carrier. In a particular embodiment said SARI is selected from the group consisting of: citalopram, escitalopram, fluoxetine, fluvoxamine, paroxetine, and sertraline or mixtures thereof and said tricyclic is selected from the group consisting of: amitriptyline, clomipramine, dosulepin dothiepin, doxepin, maprotiline, mianserin, trazodone, trimipramine, amoxapine, imipramine, lofepramine, and nortriptyline or mixtures thereof.

In a further aspect the present invention provides the use of HPPD inhibitors in the treatment of withdrawal symptoms associated with an addictive drug which causes dopamine dependent associative learning disorders. Such addictive drugs include: psychostimulants like cocaine and amphetamine and also narcotic analgesics, opiate, nicotine, ethanol, tetrahydrocannabinol and phencyclidine.

Nicotine is a stimulant which temporarily improves alertness and memory, but also forms a strong physical and psychological chemical dependence (addiction). Through the use of cigarettes, cigars, and chewing tobacco, nicotine is one of the most heavily used addictive drugs.

Medical research has determined that smoking is a major contributing factor towards many health problems, particularly lung cancer, emphysema, and cardiovascular disease. Many of tobacco's health effects can be minimised through smoking cessation.

Many smokers wish to quit smoking but need pharmacotherapy to assist them as dependence on nicotine is difficult to treat. Pharmacological options for the treatment of tobacco dependence are currently limited to nicotine replacement therapy and, more recently, sustained-release bupropion which *per se* can have limited effectiveness.

The present invention provides for the use of a HPPD inhibitor to act as, *inter alia,* a stimulant to increase production of brain dopamine. This stimulant effect will be exploited as an aid to achieving abstinence from tobacco containing products by reducing craving, lowering the brain reward-threshold and alleviating some nicotine withdrawal symptoms, which may include depressed mood, irritability, difficulty concentrating and increased appetite.

Cocaine is an example of another addictive drug. Cocaine is a stimulant, creating what has been described as a "euphoric sense of happiness" and "increased energy". Cocaine can cause physical and psychological dependence, making withdrawal difficult. Cocaine has become the second most widely used recreational drug in the U.S.

There are currently no approved or effective pharmacological agents to help patients addicted to cocaine, withdraw from their habit. The severity of withdrawal symptoms is one of the major reasons for patients failing to withdraw. The invention provides, *inter alia,* the use a HPPD inhibitor to increase the brain concentration or turnover of dopamine, and so reduce the adverse symptoms of cocaine withdrawal, and increase the likelihood of successful withdrawal.

The present invention therefore also seeks to provide, *inter alia*, a pharmaceutical for use in the treatment of the withdrawal symptoms associated with an addictive drug which cause dopamine dependant associative learning disorders, which pharmaceutical overcomes and/or ameliorates the problems mentioned herein.

Accordingly the present invention provides, amongst other things, compositions and methods for their use to inhibit 4-hydroxyphenylpyruvate dioxygenase in animals such that an increase in dopamine synthesis and/or turnover is achieved.

The present invention therefore provides the use of at least one compound capable of inhibiting 4-hydroxyphenylpyruvate dioxygenase (HPPD) in an animal in the manufacture of a medicament for use in the treatment of the withdrawal symptoms associated with an addictive drug which causes dopamine dependant associative learning disorders in said animal. In a particular embodiment said addictive drug is a drug selected from the group consisting of: cocaine, amphetamine, opiate, nicotine, ethanol, tetrahydrocannabinol and phencyclidine. In a particular embodiment said drug is nicotine. In a further embodiment said drug is cocaine.

The present invention still further provides the use as described above wherein said medicament comprises a compound selected from the group depicted as compound 1; 2; and 3.22.

The present invention still further provides the use as described above wherein the medicament comprises a further compound which is also capable of inhibiting 4-hydroxyphenylpyruvate dioxygenase (HPPD) in an animal.

The present invention still further provides the use as described above wherein said medicament comprises a nicotine replacement therapy. Such nicotine replacement therapies are well known in the art and include gums, patches, tablets, dragees, sprays and inhalers.

The present invention still further provides the use as described above wherein said medicament comprises bupropion.

The present invention still further provides the use as described above wherein said medicament is used in conjunction with a nicotine replacement therapy.

The present invention still further provides the use as described above wherein said medicament is used in conjunction with bupropion.

In a particular embodiment said nicotine replacement therapy may be administered separately to the medicament comprising said HPPD inhibitor.

In a particular embodiment said buproprion may be administered separately to the medicament comprising said HPPD inhibitor.

In a still further aspect the present invention provides a kit comprising a pharmaceutically effective amount of compound selected from the group consisting of: 1; 3.22; and 2 or a pharmaceutically acceptable salt thereof and a pharmaceutically effective amount of a nicotine replacement therapy and/or bupropion and a means for the delivery thereof to an animal.

In a still further aspect the present invention provides a pharmaceutical composition comprising as an active ingredient a pharmaceutically effective amount of a compound selected from the group consisting of: compound 1; 3.22; and 2 or a pharmaceutically acceptable salt thereof and a pharmaceutically effective amount of a nicotine replacement therapy and/or bupropion together with a pharmaceutically acceptable diluent or carrier.

The present invention still further provides a pharmaceutical composition as described above which is in a form suitable for oral or parenteral administration.

The present invention still further provides a pharmaceutical composition as described above which is in palatable form suitable for oral administration selected from the group consisting of: tablets; lozenges; hard capsules; aqueous suspensions; oily suspensions; emulsions; dispersible powders; dispersible granules; syrups and elixirs.

The present invention still further provides a pharmaceutical composition as described above which is intended for oral use and is in the form of hard or soft gelatin capsules.

The present invention still further provides a pharmaceutical composition as described above which is in a form suitable for parenteral administration.

Thus, the compositions of such HPPD inhibitors for use in the invention may be in various conventional forms well know in the pharmaceutical art and which are especially adapted for pharmaceutical purposes that is for administration to man and other warm-blooded animals.

The compositions of the invention may be obtained by conventional procedures using conventional pharmaceutical excipients, well known in the art.

Thus, compositions intended for oral use will normally contain, for example, at least one or more colouring, sweetening, flavouring and/or preservative agents and may be in the form of hard gelatin capsules in which the active ingredient is mixed with an inert solid diluent, for example, calcium carbonate, calcium phosphate or kaolin, Compositions for oral use may also be in the form of soft gelatin capsules in which the active ingredient is mixed with water or an oil such as arachis oil, liquid paraffin or olive oil.

Suitable pharmaceutically acceptable excipients for use in tablet formulations include, for example, inert diluents such as lactose, sodium carbonate, calcium phosphate or calcium carbonate, granulating and disintegrating agents such as corn starch or alginic acid; binding agents such as gelatin or starch; lubricating agents such as magnesium stearate, stearic acid or talc; preservative agents such as ethyl or propyl p-hydroxybenzoate, and anti-oxidants, such as ascorbic acid.

Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Aqueous suspensions will generally contain the active ingredient in finely powdered form together with one or more pharmaceutically acceptable suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxyethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan mono-oleate.

Aqueous suspensions will also typically contain one or more preservatives (such as ethyl or propyl p-hydroxybenzoate, anti-oxidants (such as ascorbic acid), colouring agents, normally together with a flavouring and/or sweetening agent (such as sucrose, saccharin or aspartame).

Oily suspensions may be formulated by suspending the active ingredient in a vegetable oil (such as arachis oil, olive oil, sesame oil or coconut oil) or in a mineral oil (such as liquid paraffin).

The oily suspensions may also contain a thickening agent such as beeswax, hard paraffin or cetyl alcohol.

Sweetening agents such as those set out above, and flavouring agents may be added to provide a palatable oral preparation.

These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives.

Suitable dispersing or wetting agents and suspending agents are exemplified by those already mentioned above.

Additional pharmaceutically acceptable excipients such as sweetening, flavouring and colouring agents, will generally also be present.

The pharmaceutical compositions of the invention may also be in the form of oil-in-water emulsions.

The oily phase may be a vegetable oil, such as olive oil or arachis oil, or a mineral oil, such as for example liquid paraffin or a mixture of any of these.

Suitable emulsifying agents may be, for example, naturally-occurring gums such as gum acacia or gum tragacanth, naturally-occurring phosphatides such as soya bean, lecithin, or esters or partial esters derived from fatty acids and hexitol anhydrides (for example sorbitan monooleate) and condensation products of the said partial esters with ethylene oxide such as polyoxyethylene sorbitan monooleate.

The emulsions may also contain sweetening, flavouring and preservative agents.

Syrups and elixirs may be formulated with sweetening agents such as glycerol, propylene glycol, sorbitol, aspartame or sucrose, and may also contain a demulcent, preservative, flavouring and/or colouring agent.

The pharmaceutical compositions may also be in the form of a sterile injectable aqueous or oily suspension, which may be formulated according to known procedures using one or more of the appropriate dispersing or wetting agents and suspending agents, which have been mentioned above.

A sterile injectable preparation may also be a sterile injectable solution or suspension in a non-toxic parenterally-acceptable diluent or solvent, for example a solution in 1,3-butanediol.

### Dosage

The amount of active ingredient that is combined with one or more excipients to produce a single dosage form will necessarily vary depending upon the host treated and the particular route of administration.

Generally, a formulation intended for oral administration to humans will generally contain for example from 0.01mg to 10mg of active agent per Kg of bodyweight combined with an appropriate and convenient amount of excipients.

Dosage unit forms will generally contain about 0.1 mg to about 500 mg of an active ingredient.

More specifically, a formulation comprising compound 2, for example, intended for oral administration to humans will generally contain for example from 0.01mg to 1mg of active agent per Kg of bodyweight combined with an appropriate and convenient amount of excipients.

Dosage unit forms for a formulation comprising compound 2 will generally contain about 0.1 mg to about 100 mg of an active ingredient.

However, it will be readily understood that it may be necessary to vary the dose of the active ingredient administered in accordance with well known medical practice in order to take account of the nature and severity of the condition or disease under treatment, any concurrent therapy, and of the age, weight, genotype and sex of the patient receiving treatment.

Generally, in therapeutic use, it is envisaged that a composition according to the invention would be administered so that a dose of the HPPD inhibitor (or of an equivalent amount of a pharmaceutically acceptable salt thereof) is received which is generally in the range 0.00002 to 10 mg/kg/day, or 0.001 to 500 mg/day more specifically, 0.05-10mg/day and 0.1-5mg/day or 0.01 to 10 mg of active agent per Kg of bodyweight daily given if necessary in divided doses.

More specifically, for a composition comprising compound 3.22 or 2, in therapeutic use, it is envisaged that a composition according to the invention would be administered so that a dose of the HPPD inhibitor (or of an equivalent amount of a pharmaceutically acceptable salt thereof) is received which is generally in the range 0.0002 to 1 mg/kg/day, or 0.01 to 100 mg/day. More specifically, from between 0.05 to 10mg/day and 0.1 to 5mg/day or 0.01 to 1mg of active agent per Kg of bodyweight daily given if necessary in divided doses. All ranges throughout this specification are inclusive. For example from 0.01 to 100 includes the values 0.01 and 100.

Intermittent dosing of the HPPD inhibitor (or of a pharmaceutically acceptable salt thereof) may also be desirable.

In addition to assessment of the overall condition of the patient, the effects of administration of the HPPD inhibitor thereof may be monitored by standard clinical chemical and blood assays.

In a still further aspect of the invention there is provided a method of treating and/or preventing depression comprising administering to an animal a pharmaceutically effective amount of a compound selected from the group consisting of: compound 1; 2; and 3.22 or a composition as described above.

In a still further aspect of the invention there is provided a method of treating an animal suffering from withdrawal symptoms resulting from addiction to a drug which is responsible for the development of dopamine dependant associative learning disorders in said animal comprising administering to said animal a pharmaceutically effective amount of a compound selected from the group consisting of: compound 1; 3.22; and 2 or a composition as described above.

In a preferred embodiment of the invention said animal is a human being.

HPPD inhibitors that are applicable to the present invention include compounds of formula I (the term formula I may be interchanged with compound 1): 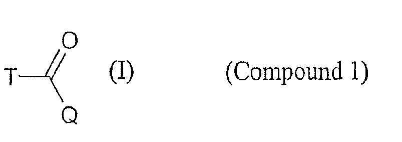 wherein;
T is T₁ 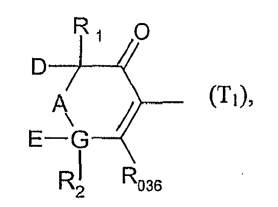 Wherein:
G is C or N wherein when G is N then only one of E and R₂ are present;
D is hydrogen or R₃;
E is hydrogen or R₄; or
D and E together are C₂-C₃alkylene which can be mono- or poly-substituted by R₆;
A is C₁-C₂alkylene which can be mono- or poly-substituted by R₅; or A may additionally be carbonyl, oxygen or -N-R₇- when D and E are other than C₂-C₃alkylene;
R₁, R₂, R₃, R₄, R₅ and R₆ are each independently of the others hydrogen, C₁-C₄alkyl, phenyl, C₁-C₄alkoxy, halogen, hydroxy, cyano, hydroxycarbonyl or C₁-C₄alkoxycarbonyl;
or R₂ and R₄ together form a C₂-C₄alkylene chain which can be interrupted by oxygen and/or carbonyl and/or sulfur, with the proviso that the oxygen and sulfur atoms are separated by at least one methylene group;
R₇ is C₁-C₄alkyl, alkoxycarbonyl or C₁-C₄alkylcarbonyl;
R₀₃₆ is hydroxy, O⁻M⁺, wherein M⁺ is an alkali metal cation or ammonium cation, halogen, C₁-C₁₂alkylsulfonyloxy, amino, C₁-C₄alkylthio, C₁-C₁₂alkylsulfinyl, C₁-C₁₂alkylsulfonyl, C₁-C₁₂haloalkylthio, C₁-C₁₂haloalkylsulfinyl, C₁-C₁₂haloalkylsulfonyl, C₁-C₆alkoxy-C₁-C₆alkylthio, C₁-C₆alkoxy-C₁-C₆alkylsulfinyl, C₁-C₆alkoxy-C₁-C₆alkylsulfonyl, C₃-C₁₂alkenylthio, C₃-C₁₂alkenylsulfinyl, C₃-C₁₂alkenylsulfonyl, C₃-C₁₂alkynylthio, C₃-C₁₂alkynylsulfinyl, C₃-C₁₂alkynylsulfonyl, C₁-C₄alkoxycarbonyl-C₁-C₄alkylthio, C₁-C₄alkoxycarbonyl-C₁-C₄alkylsulfinyl, C₁-C₄alkoxycarbonyl-C₁-C₄alkylsulfonyl, (C₁-C₄alkoxy)₂P(O)O, C₁-C₄alkyl-(C₁-C₄alkoxy)P(O)O, H(C₁-C₄alkoxy)P(O)O, R₀₃₇R₀₃₈N, R₀₃₉R₀₄₀NNH, R₀₄₁R₀₄₂NC(O)O-, R₀₄₃R₀₄₄NC(O)NH-, C₁-C₁₈alkylcarbonyloxy, C₂-C₁₈alkenylcarbonyloxy, C₂-C₁₈alkynylcarbonyloxy, C₃-C₆cycloalkylcarbonyloxy, C₁-C₁₂alkoxycarbonyloxy, C₁-C₁₂alkylthiocarbonyloxy or C₁-C₁₂alkylthiocarbamoyl, wherein the alkyl, alkenyl and alkynyl groups can be substituted by halogen, C₁-C₆alkoxy, C₁-C₆alkylthio, C₁-C₆alkylsulfinyl, C₁-C₆alkylsulfonyl or by cyano; or
R₀₃₆ is phenoxy, phenylthio, phenylsulfinyl, phenylsulfonyl, phenylsulfonylamino, phenylsulfonyloxy, benzoyloxy or benzoyl-C₁-C₆alkoxy, wherein the phenyl groups can in turn be substituted one or more times by halogen, nitro, cyano, C₁-C₄alkyl, C₁-C₄haloalkyl, C₁-C₄alkoxy and/or C₁-C₄haloalkoxy,
or R₀₃₆ is a group Het₀₇-thio, Het₀₈-sulfinyl, Het₀₉-sulfonyl, Het₀₁₀-(CO)O or Het₀₁₁-N(R₀₄₇); wherein
Het₀₇, Het₀₈, Het₀₉, Het₀₁₀ and Het₀₁₁ are each independently of the others a five- to ten-membered monocyclic or annellated bicyclic ring system which may be aromatic or partially saturated and may contain from 1 to 4 hetero atoms selected from nitrogen, oxygen and sulfur, and each ring system may contain not more than two oxygen atoms and not more than two sulfur atoms, and the ring system itself can be substituted by C₁-C₆alkyl, C₁-C₆haloalkyl, C₁-C₆alkoxy, C₁-C₆haloalkoxy, C₁-C₆alkylthio, C₁-C₆alkylsulfinyl, C₁-C₆alkylsulfonyl, di(C₁-C₄alkyl)aminosulfonyl, di(C₁-C₄alkyl)amino, halogen, cyano, nitro or by phenyl, and the substituents on the nitrogen atom in the heterocyclic ring are other than halogen;
R₀₃₇, R₀₃₈, R₀₃₉, R₀₄₀, R₀₄₁, R₀₄₂, R₀₄₃, R₀₄₄ and R₀₄₇ are each independently of the others hydrogen or C₁-C₆alkyl; or
R₀₃₇ and R₀₃₈ together or R₀₃₉ and R₀₄₀ together or R₀₄₁ and R₀₄₂ together or R₀₄₃ and R₀₄₄ together are pyrrolidino, piperidino, morpholino or thiomorpholino, which can be mono- or poly-substituted by methyl groups;
or T is T₂ 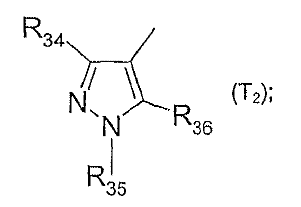 wherein
R₃₄ is hydrogen, C₁-C₄alkyl, C₁-C₄haloalkyl, C₃-C₆cycloalkyl, C₂-C₄alkenyl, C₂-C₄alkynyl or benzyl, it being possible for the phenyl group to be substituted one or more times by C₁-C₆alkyl, C₁-C₆haloalkyl, C₁-C₆alkoxy, C₁-C₆haloalkoxy, halogen, cyano, hydroxy and/or nitro;
R₃₅ is hydrogen, C₁-C₄alkyl, C₁-C₄haloalkyl, C₃-C₆cycloalkyl, C₃-C₄alkenyl, C₃-C₄alkynyl or benzyl, it being possible for the phenyl group to be substituted one or more times by C₁-C₆alkyl, C₁-C₆haloalkyl, C₁-C₆alkoxy, C₁-C₆haloalkoxy, halogen, cyano, hydroxy and/or nitro;
R₃₆ is hydroxy, O⁻M⁺, wherein M⁺ is an alkali metal cation or ammonium cation, halogen, C₁-C₁₂alkylsulfonyloxy, amino, C₁-C₄alkylthio, C₁-C₁₂alkylsulfinyl, C₁-C₁₂alkylsulfonyl, C₁-C₁₂haloalkylthio, C₁-C₁₂haloalkylsulfinyl, C₁-C₁₂haloalkylsulfonyl, C₁-C₆alkoxy-C₁-C₆alkylthio, C₁-C₆alkoxy-C₁-C₆alkylsulfinyl, C₁-C₆alkoxy-C₁-C₆alkylsulfonyl, C₃-C₁₂alkenylthio, C₃-C₁₂alkenylsulfinyl, C₃-C₁₂alkenylsulfonyl, C₃-C₁₂alkynylthio, C₃-C₁₂alkynylsulfinyl, C₃-C₁₂alkynylsulfonyl, C₁-C₄alkoxycarbonyl-C₁-C₄alkylthio, C₁-C₄alkoxycarbonyl-C₁-C₄alkylsulfinyl, C₁-C₄alkoxycarbonyl-C₁-C₄alkylsulfonyl, (C₁-C₄alkoxy)₂P(O)O, C₁-C₄alkyl-(C₁-C₄alkoxy)P(O)O, H(C₁-C₄alkoxy)P(O)O, R₃₇R₃₈N, R₃₉R₄₀NNH, R₄₁R₄₂NC(O)O-, R₄₃R₄₄NC(O)NH-, C₁-C₁₈alkylcarbonyloxy, C₂-C₁₈alkenylcarbonyloxy, C₂-C₁₈alkynylcarbonyloxy, C₃-C₆-cycloalkylcarbonyloxy, C₁-C₁₂alkoxycarbonyloxy, C₁-C₁₂alkylthiocarbonyloxy or C₁-C₁₂alkylthiocarbamoyl, wherein the alkyl, alkenyl and alkynyl groups can be substituted by halogen, C₁-C₆alkoxy, C₁-C₆alkylthio, C₁-C₆alkylsulfinyl, C₁-C₆alkylsulfonyl or by cyano; or
R₃₆ is phenoxy, phenylthio, phenylsulfinyl, phenylsulfonyl, phenylsulfonylamino, phenylsulfonyloxy, benzoyloxy or benzoyl-C₁-C₆alkoxy, it being possible for the phenyl groups in turn to be substituted one or more times by halogen, nitro, cyano, C₁-C₄alkyl, C₁-C₄haloalkyl, C₁-C₄alkoxy and/or C₁-C₄haloalkoxy,
or R₃₆ is a group Het₇-thio, Het₈-sulfinyl, Het₉-sulfonyl, Het₁₀-(CO)O or Het₁₁-N(R₄₇); wherein
Het₇, Het₈, Het₉, Het₁₀ and Het₁₁ are each independently of the others a five- to ten-membered monocyclic or annellated bicyclic ring system which may be aromatic or partially saturated and may contain from 1 to 4 hetero atoms selected from nitrogen, oxygen and sulfur, and each ring system may contain not more than two oxygen atoms and not more than two sulfur atoms, and the ring system itself can be substituted by C₁-C₆alkyl, C₁-C₆haloalkyl, C₁-C₆alkoxy, C₁-C₆haloalkoxy, C₁-C₆alkylthio, C₁-C₆alkylsulfinyl, C₁-C₆alkylsulfonyl, di(C₁-C₄alkyl)aminosulfonyl, di(C₁-C₄alkyl)amino, halogen, cyano, nitro or by phenyl, and the substituents on the nitrogen atom in the heterocyclic ring are other than halogen;
R₃₇, R₃₈, R₃₉, R₄₀, R₄₁, R₄₂, R₄₃, R₄₄ and R₄₇ are each independently of the others hydrogen or C₁-C₆alkyl; or
R₃₇ and R₃₈ together or R₃₉ and R₄₀ together or R₄₁ and R₄₂ together or R₄₃ and R₄₄ together are pyrrolidino, piperidino, morpholino or thiomorpholino, which can be mono- or poly-substituted by methyl groups;
or T is T₃ 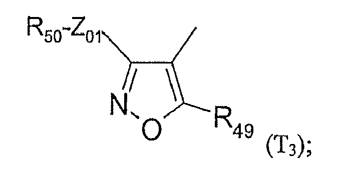 wherein
R₄₉ is C₁-C₄alkyl, C₁-C₄haloalkyl, C₃-C₆cycloalkyl or halo-substituted C₃-C₆cycloalkyl; Z₀₁ is a chemical bond, S, SO or SO₂; or -CO₂-
R₅₀ is hydrogen or C₁-C₃alkylene which can be substituted by the following substituents: halogen, hydroxy, C₁-C₆alkoxy, C₂-C₆alkenyl, C₂-C₆alkynyl, C₃-C₆cycloalkyl, C₁-C₆alkoxy-C₁-C₆alkoxy, C₁-C₆alkoxy-C₁-C₆alkoxy-C₁-C₆alkoxy, (3-oxetanyl)-oxy, C₁-C₆alkyl-substituted (3-oxetanyl)-oxy, benzylthio, benzylsulfinyl, benzylsulfonyl, phenyl, phenoxy, phenylthio, phenylsulfinyl or phenylsulfonyl, it being possible for the phenyl- and benzyl-containing groups in turn to be substituted by one or more C₁-C₆alkyl, C₁-C₆haloalkyl, C₁-C₆alkoxy, C₁-C₆haloalkoxy, halogen, cyano, hydroxy and/or nitro groups;
or R₅₀ is phenyl, it being possible for the phenyl-containing group in turn to be substituted by one or more C₁-C₆alkyl, C₁-C₆haloalkyl, C₁-C₆alkoxy, C₁-C₆haloalkoxy, halogen, cyano, hydroxy and/or nitro groups,
or R₅₀ is C₃-C₆cycloalkyl, C₁-C₆alkoxy- or C₁-C₆alkyl-substituted C₃-C₆cycloalkyl, 3-oxetanyl or C₁-C₆alkyl-substituted 3-oxetanyl;
or T is T₄ 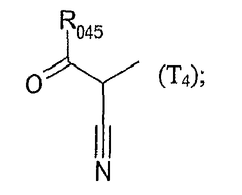 wherein
R₀₄₅ is C₁-C₆alkyl, C₁-C₆haloalkyl, C₃-C₆cycloalkyl or halo-substituted C₃-C₆cycloalkyl; and their pharmaceutically acceptable salts, isomers and enantiomers.

The compounds of formula I also include the salts which such compounds are able to form with amines, alkali metal and alkaline earth metal bases or quaternary ammonium bases. Among the alkali metal and alkaline earth metal hydroxides as salt formers, special mention should be made of the hydroxides of lithium, sodium, potassium, magnesium and calcium, but especially the hydroxides of sodium and potassium.

Examples of amines suitable for ammonium salt formation include ammonia as well as primary, secondary and tertiary C₁-C₁₈alkylamines, C₁-C₄hydroxyalkylamines and C₂-C₄alkoxyalkylamines, for example methylamine, ethylamine, n-propylamine, isopropylamine, the four butylamine isomers, n-amylamine, isoamylamine, hexylamine, heptylamine, octylamine, nonylamine, decylamine, pentadecylamine, hexadecylamine, heptadecylamine, octadecylamine, methylethylamine, methylisopropylamine, methylhexylamine, methylnonylamine, methylpentadecylamine, methyloctadecylamine, ethylbutylamine, ethylheptylamine, ethyloctylamine, hexylheptylamine, hexyloctylamine, dimethylamine, diethylamine, di-n-propylamine, diisopropylamine, din-butylamine, di-n-amylamine, diisoamylamine, dihexylamine, diheptylamine, dioctylamine, ethanolamine, n-propanolamine, isopropanolamine, N,N-diethanolamine, N-ethylpropanolamine, N-butylethanolamine, allylamine, n-butenyl-2-amine, n-pentenyl-2-amine, 2,3-dimethylbutenyl-2-amine, dibutenyl-2-amine, n-hexenyl-2-amine, propylenediamine, trimethylamine, triethylamine, tri-n-propylamine, triisopropylamine, tri-n-butylamine, triisobutylamine, tri-sec-butylamine, tri-n-amylamine, methoxyethylamine and ethoxyethylamine; heterocyclic amines, for example pyridine, quinoline, isoquinoline, morpholine, piperidine, pyrrolidine, indoline, quinuclidine and azepine; primary arylamines, for example anilines, methoxyanilines, ethoxyanilines, o-, m- and p-toluidines, phenylenediamines, benzidines, naphthylamines and o-, m- and p-chloroanilines; but especially triethylamine, isopropylamine and diisopropylamine.

Because the compounds of formula I wherein T is T₁ are preferably in enolised forms or in the form of salts, formula I also includes the enolised forms of formulae Ia, Ib, Ic and Id wherein M is hydrogen or a metal ion or an ammonium ion.

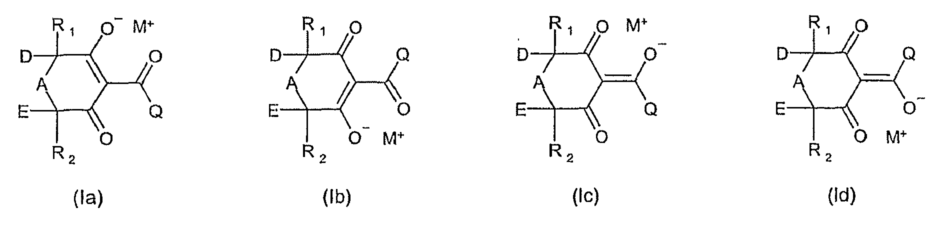

Since compounds of formula I may also contain asymmetric carbon atoms, for example in the case of the carbon atom carrying R₁, D and A, all stereoisomeric forms are also included.

The organic substituent Q may be an inert substituent of any desired structure, provided that the compounds of formula I retain their action as HPPD inhibitors in animals. Such tests of these compounds may be carried out in accordance with the experimental methods described herein.

Q is preferably a mono- or poly-substituted phenyl, pyridyl or heteroaryl group, especially 2-benzoyl, 2-isonicotinoyl and 2-nicotinoyl derivatives, the substitution pattern of those groups being freely selectable provided that the compounds of formula I retain their action as HPPD inhibitors in animals.

In a particular embodiment said HPPD inhibitors are compounds of formula I
wherein
Q is Q₁ 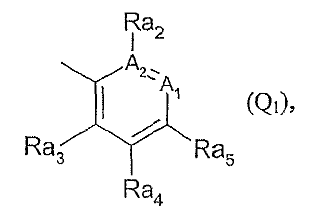 wherein
A₁ or A₂ are independently selected from methine, C(Ra₁) or N(O)ₚ; (wherein preferably at least one of A₁ or A₂ is methine
p is 0 or 1;
Ra₁ is hydrogen, C₁-C₆alkyl, hydroxy, C₁-C₆alkoxy, C₁-C₆haloalkoxy, C₃-C₆alkenyloxy, C₃-C₆haloalkenyloxy, C₃-C₆alkynyloxy, C₁-C₄alkylcarbonyloxy, C₁-C₄alkylsulfonyloxy, tosyloxy, C₁-C₄alkylthio, C₁-C₄alkylsulfinyl, C₁-C₄alkylsulfonyl, C₁-C₄alkylamino, di-C₁-C₄alkylamino, C₁-C₄alkoxycarbonyl, C₁-C₄haloalkyl, formyl, cyano, halogen, phenyl or phenoxy; it being possible for phenyl in turn to be substituted by C₁-C₃alkyl, C₁-C₃haloalkyl, C₁-C₃alkoxy, C₁-C₃haloalkoxy, halogen, cyano or by nitro; or Ra₁ is a three- to ten-membered monocyclic ring system or, together with Ra₂ or Ra₅, annellated mono- or bi-cyclic ring system which may be interrupted by oxygen, sulfur, SO, SO₂, NRa₆, carbonyl and/or by =NORa₇, the ring system, unless it is annellated, being bonded to the carbon atom of the substituent A₁ directly or by way of a C₁-C₄alkylene, -CH=CH-, -C≡C-, -CH₂O-, -CH₂N(C₁-C₄alkyl)-, -CH₂S-, -CH₂SO- or - CH₂SO₂- group, and the ring system may contain not more than two oxygen atoms and not more than two sulfur atoms, and the ring system can itself be mono-, di- or tri-substituted by C₁-C₆alkyl, C₁-C₆haloalkyl, C₂-C₆alkenyl, C₂-C₆haloalkenyl, C₂-C₆alkynyl, C₂-C₆haloalkynyl, C₁-C₆alkoxy, C₁-C₆haloalkoxy, C₃-C₆alkenyloxy, C₃-C₆alkynyloxy, C₁-C₆alkylthio, C₁-C₆haloalkylthio, C₃-C₆alkenylthio, C₃-C₆haloalkenylthio, C₃-C₆alkynylthio, C₁-C₄alkoxy-C₁-C₂alkylthio, C₁-C₄alkylcarbonyl-C₁-C₂alkylthio, C₁-C₄alkoxycarbonyl-C₁-C₂alkylthio, cyano-C₁-C₄alkylthio, C₁-C₆alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, di(C₁-C₂alkyl)aminosulfonyl, di(C₁-C₄alkyl)amino, halogen, cyano, nitro, phenyl and/or benzylthio, it being possible for phenyl and benzylthio in turn to be substituted on the phenyl ring by C₁-C₃alkyl, C₁-C₃haloalkyl, C₁-C₃alkoxy, C₁-C₃haloalkoxy, halogen, cyano or by nitro, and substituents on the nitrogen atom in the heterocyclic ring are other than halogen; or Ra₁ is the group -X₅-X₇ or the group -X₆-X₅-X₇; wherein
X₆ is a C₁-C₆alkylene, C₃-C₆alkenylene or C₃-C₆alkynylene chain which can be mono- or poly-substituted by halogen and/or by X₈, the unsaturated bonds of the chain not being bonded directly to the substituent X₅;
X₈ is hydroxy, C₁-C₆alkoxy, C₃-C₆cycloalkyloxy, C₁-C₆alkoxy-C₁-C₆alkoxy, C₁-C₆alkoxy-C₁-C₆alkoxy-C₁-C₆alkoxy or C₁-C₂alkylsulfonyloxy;
X₅ is oxygen, -O(CO)-, -(CO)O-, -O(CO)O-, -N(C₁-C₄alkyl)-O-, -O-N(C₁-C₄alkyl)-, thio, sulfinyl, sulfonyl, -SO₂N(C₁-C₄alkyl)-, -N(C₁-C₄alkoxy)SO₂-, -N(C₁-C₄alkyl)SO₂-, -N(C₁-C₂alkoxy-C₁-C₂alkyl)SO₂- or -N(C₁-C₄alkyl)-;
Ra₆ is hydrogen, C₁-C₄alkyl, C₁-C₄alkylthio-C₁-C₄carbonyl, C₁-C₄alkylsulfinyl-C₁-C₄carbonyl, C₁-C₄alkylsulfonyl-C₁-C₄carbonyl, C₁-C₄alkoxycarbonyl, C₁-C₄alkylcarbonyl, phenylcarbonyl or phenyl, it being possible for the phenyl groups in turn to be substituted by C₁-C₄alkyl, C₁-C₄haloalkyl, C₁-C₄alkoxy, C₁-C₄haloalkoxy, C₁-C₄alkylcarbonyl, C₁-C₄alkoxycarbonyl, C₁-C₄alkylamino, di-C₁-C₄alkylamino, C₁-C₄alkyl-S-, C₁-C₄alkyl-SO-, C₁-C₄alkyl-SO₂, C₁-C₄alkyl-S(O)₂O, C₁-C₄haloalkyl-S-, C₁-C₄haloalkyl-SO, C₁-C₄haloalkyl-SO₂, C₁-C₄haloalkyl-S(O)₂O, C₁-C₄alkyl-S(O)₂NH, C₁-C₄alkyl-S(O)₂N(C₁-C₄alkyl), halogen, nitro or by cyano;
Ra₇ is C₁-C₄alkyl;
Ra₂ is hydrogen, C₁-C₆alkyl, C₁-C₆haloalkyl, C₂-C₆alkenyl, C₂-C₆haloalkenyl, vinyl substituted by C₁-C₂alkoxycarbonyl or by phenyl, C₂-C₆alkynyl, C₂-C₆haloalkynyl, ethynyl substituted by trimethylsilyl, hydroxy, C₁-C₂alkoxy, C₁-C₂alkoxycarbonyl or by phenyl, C₃-C₆allenyl, C₃-C₆cycloalkyl, halo-substituted C₃-C₆cycloalkyl, C₁-C₆alkoxy, C₃-C₆alkenyloxy, C₃-C₆alkynyloxy, C₁-C₆haloalkoxy, C₃-C₆haloalkenyloxy, cyano-C₁-C₄alkoxy, C₁-C₄alkoxy-C₁-C₄alkoxy, C₁-C₄alkylthio-C₁-C₄alkoxy, C₁-C₄alkylsulfinyl-C₁-C₄alkoxy, C₁-C₄alkylsulfonyl-C₁-C₄alkoxy, C₁-C₄alkoxycarbonyl-C₁-C₄alkoxy, C₁-C₆alkylthio, C₁-C₆alkylsulfinyl, C₁-C₆alkylsulfonyl, C₁-C₆haloalkylthio, C₁-C₆haloalkylsulfinyl, C₁-C₆haloalkylsulfonyl, C₁-C₄alkoxycarbonyl-C₁-C₄alkylthio, C₁-C₄alkoxycarbonyl-C₁-C₄alkylsulfinyl, C₁-C₄alkoxycarbonyl-C₁-C₄alkylsulfonyl, benzyl-S-, benzyl-SO-, benzyl-SO₂-, C₁-C₆alkylamino, di-C₂-C₆alkylamino, C₁-C₆alkylaminosulfonyl, di(C₁-C₆alkylamino)sulfonyl, benzyloxy, benzyl, phenyl, phenoxy, phenylthio, phenylsulfinyl or phenylsulfonyl, it being possible for the phenyl-containing groups in turn to be substituted by C₁-C₃alkyl, C₁-C₃haloalkyl, C₁-C₃alkoxy, C₁-C₃haloalkoxy, halogen, cyano or by nitro, or Ra₂ is OS-C₁-C₄alkyl, OSO-C₁-C₄alkyl, OSO₂-C₁-C₄alkyl, OS-C₁-C₄haloalkyl, OSO-C₁-C₄haloalkyl, OSO₂-C₁-C₄haloalkyl, N(C₁-C₄alkyl)-S-C₁-C₄alkyl, N(C)-C₄alkyl)-SO-C₁-C₄alkyl, N(C₁-C₄alkyl)-SO₂-C₁-C₄alkyl, cyano, carbamoyl, C₁-C₄alkoxycarbonyl, formyl, halogen, rhodano, amino, hydroxy-C₁-C₄alkyl, C₁-C₄alkoxy-C₁-C₄alkyl, C₁-C₄alkyl-S-C₁-C₄alkyl, C₁-C₄alkyl-SO-C₁-C₄alkyl, C₁-C₄alkyl-SO₂-C₁-C₄alkyl, cyano-C₁-C₄alkyl, C₁-C₆alkylcarbonyloxy-C₁-C₄alkyl, C₁-C₄alkoxycarbonyl-C₁-C₄alkyl, C₁-C₄alkoxycarbonyloxy-C₁-C₄alkyl, C₁-C₄rhodano-C₁-C₄alkyl, benzoyloxy-C₁-C₄alkyl, C₂-C₆oxiranyl, C₁-C₄alkylamino-C₁-C₄alkyl, die C₁-C₄alkyl)amino-C₁-C₄alkyl, C₁-C₁₂alkylthiocarbonyl-C₁-C₄alkyl or formyl-C₁-C₄alkyl, or Ra₂ is a five- to ten-membered monocyclic or annellated bicyclic ring system which may be aromatic or partially saturated and may contain from 1 to 4 hetero atoms selected from nitrogen, oxygen and sulfur, the ring system being bonded to the pyridine ring by way of a C₁-C₄alkylene, -CH=CH-, -C≡C-, -CH₂O-, -CH₂N(C₁-C₄alkyl)-, -CH₂SO- or-CH₂SO₂-group, and each ring system may contain not more than two oxygen atoms and not more than two sulfur atoms, and the ring system itself can be mono-, di- or tri-substituted by C₁-C₆alkyl, C₁-C₆haloalkyl, C₃-C₆alkenyl, C₃-C₆haloalkenyl, C₃-C₆alkynyl, C₃-C₆haloalkynyl, C₁-C₆alkoxy, C₁-C₆haloalkoxy, C₃-C₆alkenyloxy, C₃-C₆alkynyloxy, mercapto, C₁-C₆alkylthio, C₁-C₆haloalkylthio, C₃-C₆alkenylthio, C₃-C₆haloalkenylthio, C₃-C₆alkynylthio, C₂-C₅alkoxyalkylthio; C₃-C₅acetylalkylthio, C₃-C₆alkoxycarbonylalkylthio, C₂-C₄cyanoalkylthio, C₁-C₆alkylsulfinyl, C₁-C₆-haloalkylsulfinyl, C₁-C₆alkylsulfonyl, C₁-C₆haloalkylsulfonyl, aminosulfonyl, C₁-C₂alkylaminosulfonyl, di(C₁-C₂alkyl)aminosulfonyl, di(C₁-C₄alkyl)amino, halogen, cyano, nitro, phenyl and/or by benzylthio, it being possible for phenyl and benzylthio in turn to be substituted on the phenyl ring by C₁-C₃alkyl, C₁-C₃haloalkyl, C₁-C₃alkoxy, C₁-C₃haloalkoxy, halogen, cyano or by nitro, and substituents on the nitrogen atom in the heterocyclic ring are other than halogen;
or Ra₂ is the group -X₁-X₃ or the group -X₂-X₁-X₃; wherein
X₂ is a C₁-C₆alkylene, C₃-C₆alkenylene or C₃-C₆alkynylene chain which can be mono- or poly-substituted by halogen or by X₄, the unsaturated bonds of the chain not being bonded directly to the substituent X₁;
X₄ is hydroxy, C₁-C₆alkoxy, C₃-C₆cycloalkyloxy, C₁-C₆alkoxy-C₁-C₆alkoxy, C₁-C₆alkoxy-C₁-C₆alkoxy-C₁-C₆alkoxy or C₁-C₂alkylsulfonyloxy;
X₁ is oxygen, -O(CO)-, -(CO)O-, -O(CO)O-, -N(C₁-C₄alkyl)-O-, -O-N(C₁-C₄alkyl)-, thio, sulfinyl, sulfonyl, -SO₂N(C₁-C₄alkyl)-, -N(C₁-C₄alkyl)SO₂-, -N(C₁-C₂alkoxy-C₁-C₂alkyl)SO₂- or -N(C₁-C₄alkyl)-;
X₃ and X₇ are each independently of the other a C₁-C₈alkyl, C₃-C₆alkenyl or C₃-C₆alkynyl group which is mono- or poly-substituted by the following substituents: halogen, hydroxy, amino, formyl, nitro, cyano, mercapto, carbamoyl, C₁-C₆alkoxy, C₁-C₆alkoxycarbonyl, C₂-C₆alkenyl, C₂-C₆haloalkenyl, C₂-C₆alkynyl, C₂-C₆haloalkynyl, C₃-C₆cycloalkyl, halo-substituted C₃-C₆cycloalkyl, C₃-C₆alkenyloxy, C₃-C₆alkynyloxy, C₁-C₆haloalkoxy, C₃-C₆haloalkenyloxy, cyano-C₁-C₆alkoxy, C₁-C₆alkoxy-C₁-C₆alkoxy, C₁-C₆alkoxy-C₁-C₆alkoxy-C₁-C₆alkoxy, C₁-C₆alkylthio-C₁-C₆alkoxy, C₁-C₆alkylsulfinyl-C₁-C₆alkoxy, C₁-C₆alkylsulfonyl-C₁-C₆alkoxy, C₁-C₆alkoxycarbonyl-C₁-C₆alkoxy, C₁-C₆alkoxycarbonyl, C₁-C₆alkylcarbonyl, C₁-C₆alkylthio, C₁-C₆alkylsulfinyl, C₁-C₆-alkylsulfonyl, C₁-C₆haloalkylthio, C₁-C₆haloalkylsulfinyl, C₁-C₆haloalkylsulfonyl, oxiranyl, which can in turn be substituted by C₁-C₆alkyl, (3-oxetanyl)-oxy, which can in turn be substituted by C₁-C₆alkyl, benzylthio, benzylsulfinyl, benzylsulfonyl, C₁-C₆alkylamino, di(C₁-C₆alkyl)amino, C₁-C₄alkyl-S(O)₂O, C₁-C₄alkyl-N(C₁-C₄alkyl)SO₂-, rhodano, phenyl, phenoxy, phenylthio, phenylsulfinyl and/or phenylsulfonyl;
it being possible for the phenyl- or benzyl-containing groups in turn to be substituted by one or more C₁-C₆alkyl, C₁-C₆haloalkyl, C₁-C₆alkoxy, C₁-C₆haloalkoxy, halogen, cyano, hydroxy and/or nitro groups, or
X₃ and X₇ are each independently of the other phenyl which can be substituted one or more times by C₁-C₆alkyl, C₁-C₆haloalkyl, C₁-C₆alkoxy, C₁-C₆haloalkoxy, halogen, cyano, hydroxy and/or nitro; or
X₃ and X₇ are each independently of the other C₃-C₆cycloalkyl, C₁-C₆alkoxy- or C₁-C₆alkyl-substituted C₃-C₆cycloalkyl, 3-oxetanyl or C₁-C₆alkyl-substituted 3-oxetanyl; or X₃ and X₇ are each independently of the other a five- to ten-membered monocyclic or annellated bicyclic ring system which may be aromatic or saturated or partially saturated and may contain from 1 to 4 hetero atoms selected from nitrogen, oxygen and sulfur, the ring system being bonded to the substituent X₁ or X₅ directly or by way of a C₁-C₄alkylene, C₂-C₄alkenyl-C₁-C₄alkylene, C₂-C₄alkynyl-C₁-C₄alkylene, -N(C₁-C₄alkyl)-C₁-C₄alkylene, -SO-C₁-C₄alkylene or -SO₂-C₁-C₄alkylene group, and each ring system may contain not more than two oxygen atoms and not more than two sulfur atoms, and the ring system can itself be mono-, di- or tri-substituted by C₁-C₆alkyl, C₁-C₆haloalkyl, C₂-C₆alkenyl, C₂-C₆haloalkenyl, C₂-C₆alkynyl, C₂-C₆haloalkynyl, C₁-C₆alkoxy, hydroxy, C₁-C₆haloalkoxy, C₃-C₆alkenyloxy, C₃-C₆alkynyloxy, mercapto, C₁-C₆alkylthio, C₁-C₆haloalkylthio, C₃-C₆alkenylthio, C₃-C₆haloalkenylthio, C₃-C₆alkynylthio, C₂-C₅alkoxyalkylthio, C₃-C₅acetylalkylthio, C₃-C₆alkoxycarbonylalkylthio, C₂-C₄-cyanoalkylthio, C₁-C₆alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, di(C₁-C₂alkyl)-aminosulfonyl, di(C₁-C₄alkyl)amino, halogen, cyano, nitro, phenyl and/or by benzylthio, it being possible for phenyl and benzylthio in turn to be substituted on the phenyl ring by C₁-C₃alkyl, C₁-C₃haloalkyl, C₁-C₃alkoxy, C₁-C₃haloalkoxy, halogen, cyano or by nitro, and the substituents on the nitrogen atom in the heterocyclic ring are other than halogen; Ra₃ is hydrogen, C₁-C₆alkyl, C₁-C₆haloalkyl, C₂-C₆alkenyl, C₂-C₆haloalkenyl, C₂-C₆alkynyl, C₂-C₆haloalkynyl, C₃-C₆cycloalkyl, C₁-C₆alkoxy, C₁-C₆haloalkoxy, C₁-C₆alkylthio, C₁-C₆alkylsulfinyl, C₁-C₆alkylsulfonyl, C₁-C₆haloalkylthio, C₁-C₆haloalkylsulfinyl, C₁-C₆haloalkylsulfonyl, C₁-C₆alkylamino, di-C₂-C₆alkylamino, C₁-C₆alkylaminosulfonyl, di-C₂-C₆alkylaminosulfonyl, phenyl, phenylthio, phenylsulfinyl, phenylsulfonyl or phenoxy, it being possible for phenyl in turn to be substituted by C₁-C₃alkyl, C₁-C₃haloalkyl, C₁-C₃alkoxy, C₁-C₃haloalkoxy, halogen, cyano or by nitro, or Ra₃ is -N(C₁-C₄alkyl)-S-C₁-C₄alkyl, -N(C₁-C₄alkyl)-SO-C₁-C₄alkyl, -N(C₁-C₄alkyl)-SO₂-C₁-C₄alkyl, cyano, halogen, amino, C₁-C₄alkoxy-C₁-C₄alkyl, C₁-C₄alkyl-S-C₁-C₄alkyl, C₁-C₄alkyl-SO-C₁-C₄alkyl or C₁-C₄alkyl-SO₂-C₁-C₄alkyl;
Ra₄ is hydrogen, C₁-C₆alkyl, hydroxy, C₁-C₆alkoxy, C₁-C₆haloalkoxy, C₃-C₆alkenyloxy, C₃-C₆haloalkenyloxy, C₃-C₆alkynyloxy, C₁-C₄alkylcarbonyloxy, C₁-C₄alkylsulfonyloxy, tosyloxy, C₁-C₄alkylthio, C₁-C₄alkylsulfinyl, C₁-C₄alkylsulfonyl, C₁-C₄alkylamino, di-C₁-C₄alkylamino, C₁-C₄alkoxycarbonyl, C₁-C₄haloalkyl, formyl, cyano, halogen, phenyl or phenoxy, it being possible for phenyl in turn to be substituted by C₁-C₃alkyl, C₁-C₃haloalkyl, C₁-C₃alkoxy, C₁-C₃haloalkoxy, halogen, cyano or by nitro; or Ra₄ is a five- to ten-membered monocyclic ring system or, with Ra₃, annellated bicyclic ring system which may contain from 1 to 4 hetero atoms selected from nitrogen, oxygen and sulfur, the ring system, unless it is annellated, being bonded to the ring containing the substituent A directly or by way of a C₁-C₄alkylene, -CH=CH-, -C≡C-, - CH₂O-, -CH₂N(C₁-C₄alkyl)-, -CH₂S-, -CH₂SO- or -CH₂SO₂- group, and the ring system may contain not more than two oxygen atoms and not more than two sulfur atoms, and the ring system can itself be mono-, di- or tri-substituted by C₁-C₆alkyl, C₁-C₆haloalkyl, C₂-C₆alkenyl, C₂-C₆haloalkenyl, C₂-C₆alkynyl, C₂-C₆haloalkynyl, C₁-C₆alkoxy, C₁-C₆haloalkoxy, C₃-C₆alkenyloxy, C₃-C₆alkynyloxy, C₁-C₆alkylthio, C₁-C₆haloalkylthio, C₃-C₆alkenylthio, C₃-C₆haloalkenylthio, C₃-C₆alkynylthio, C₁-C₄alkoxy-C₁-C₂alkylthio, C₁-C₄alkylcarbonyl-C₁-C₂alkylthio, C₁-C₄alkoxycarbonyl-C₁-C₂alkylthio, cyano-C₁-C₄alkylthio, C₁-C₆alkylsulfinyl, C₁-C₆haloalkylsulfinyl, C₁-C₆alkylsulfonyl, C₁-C₆haloalkylsulfonyl, aminosulfonyl, C₁-C₂alkylaminosulfonyl, di(C₁-C₂alkyl)aminosulfonyl, di(C₁-C₄alkyl)amino, halogen, cyano, nitro, phenyl and/or by benzylthio, it being possible for phenyl and benzylthio in turn to be substituted on the phenyl ring by C₁-C₃alkyl, C₁-C₃haloalkyl, C₁-C₃alkoxy, C₁-C₃haloalkoxy, halogen, cyano or by nitro, and substituents on the nitrogen atom in the heterocyclic ring are other than halogen;
Ra₅ is hydrogen, C₁-C₆allcyl, C₁-C₆haloalkyl, C₂-C₆alkenyl, C₂-C₆haloalkenyl, C₂-C₆alkynyl, C₂-C₆haloalkynyl, C₃-C₆cycloalkyl, C₁-C₆alkoxy, C₁-C₆haloalkoxy, C₁-C₆alkylthio, C₁-C₆alkylsulfinyl, C₁-C₆alkylsulfonyl, C₁-C₆haloalkylthio, C₁-C₆haloalkylsulfinyl, C₁-C₆haloalkylsulfonyl, C₁-C₆alkylamino, di-C₂-C₆alkylamino, C₁-C₆alkylaminosulfonyl, di-C₂-C₆alkylaminosulfonyl, phenyl, phenylthio, phenylsulfinyl, phenylsulfonyl or phenoxy, it being possible for phenyl in turn to be substituted by C₁-C₃alkyl, C₁-C₃haloalkyl, C₁-C₃alkoxy, C₁-C₃haloalkoxy, halogen, cyano or by nitro, or Ra₅ is -N(C₁-C₄alkyl)-S-C₁-C₄alkyl, -N(C₁-C₄alkyl)-SO-C₁-C₄alkyl, -N(C₁-C₄alkyl)-SO₂-C₁-C₄alkyl, cyano, halogen, amino, C₁-C₄alkoxy-C₁-C₄alkyl, C₁-C₄alkyl-S-C₁-C₄alkyl, C₁-C₄alkyl-SO-C₁-C₄alkyl or C₁-C₄alkyl-SO₂-C₁-C₄alkyl, and pharmaceutically acceptable salts/N-oxides/isomers/enantiomers of those compounds.

The alkyl groups appearing in the above substituent definitions may be straight-chain or branched and are, for example, methyl, ethyl, n-propyl, isopropyl, n-butyl, sec-butyl, isobutyl or tert-butyl. Alkoxy, alkenyl and alkynyl radicals are derived from the mentioned alkyl radicals. The alkenyl and alkynyl groups may be mono- or poly-unsaturated. Alkoxy is, for example, methoxy, ethoxy, propoxy, isopropoxy, n-butoxy, isobutoxy, sec-butoxy or tert-butoxy. Alkoxycarbonyl is, for example, methoxycarbonyl, ethoxycarbonyl, propoxycarbonyl, isopropoxycarbonyl, n-butoxycarbonyl, isobutoxycarbonyl, sec-butoxycarbonyl or tert-butoxycarbonyl; preferably methoxycarbonyl or ethoxycarbonyl.

Halogen is generally fluorine, chlorine, bromine or iodine. The same is also true of halogen in conjunction with other meanings, such as haloalkyl or halophenyl.

Haloalkyl groups having a chain length of from 1 to 6 carbon atoms are, for example, fluoromethyl, difluoromethyl, trifluoromethyl, chloromethyl, dichloromethyl, trichloromethyl, 2,2,2-trifluoroethyl, 1-fluoroethyl, 2-fluoroethyl, 2-chloroethyl, 2-fluoroprop-2-yl, pentafluoroethyl, 1,1-difluoro-2,2,2-trichloroethyl, 2,2,3,3-tetrafluoroethyl and 2,2,2-trichloroethyl, pentafluoroethyl, heptafluoro-n-propyl and perfluoro-n-hexyl.

Alkenyl and alkynyl groups can be mono- or poly-unsaturated, so that alkyl, alkenyl and alkynyl chains having one or more double or triple bonds are also included. Alkenyl is, for example, vinyl, allyl, isobuten-3-yl, CH₂=CH-CH₂-CH=CH-, CH₂=CH-CH₂-CH₂-CH=CH- or CH₃-CH=CH-CH₂-CH=CH-. A preferred alkynyl is, for example, propargyl, and a preferred allenyl is CH₂=C=CH₂-.

An alkylene chain can also be substituted by one or more C₁-C₃alkyl groups, especially by methyl groups. Such alkylene chains and alkylene groups are preferably unsubstituted. The same applies also to all groups containing C₃-C₆cycloalkyl, C₃-C₅oxacycloalkyl, C₃-C₅thiacycloalkyl, C₃-C₄dioxacycloalkyl, C₃-C₄dithiacycloalkyl or C₃-C₄oxathiacycloalkyl which occur, for example, also as part of oxygen- and sulfur-containing heterocyclic ring systems of the radicals Ra₁ and Ra₂.

A C₁-C₄alkylene, C₁-C₄alkenylene or C₂-C₄alkynylene bridge which may be interrupted by oxygen, -N(C₁-C₄alkyl)-, sulfur, sulfinyl and/or sulfonyl, or in X₂ or X₆ in the meaning of a C₁-C₆alkylene, C₃-C₆alkenylene or C₃-C₆alkynylene chain which can be mono- or poly-substituted by halogen and/or by X₄ or X₈, and wherein the unsaturated bonds of the chain are not bonded directly to the substituent X₁ or X₅, is to be understood as being, for example, -CH₂-, -CH₂CH₂-, -CH₂CH₂CH₂-, -CH₂CH₂CH₂CH₂-, -CH(CH₃)-, -CH₂CH(CH₃)-, -CH₂CH(CH₃)CH₂-, -CH₂CH(Cl)CH₂-, -CH₂CH(OCH₃)CH₂-, -CH₂O-, - OCH₂-, -CH₂OCH₂-, -OCH₂CH₂-, -OCH₂CH₂CH₂-, -CH₂OCH₂CH₂-, - CH₂OCH(CH₃)CH₂-, -SCH₂-, -SCH₂CH₂-, -SCH₂CH₂CH₂-, -CH₂S-, -CH₂SCH₂-, - CH₂S(O)CH₂-, -CH₂SO₂CH₂-, -CH₂SCH₂CH₂-, -CH₂S(O)CH₂CH₂-, -CH₂SO₂CH₂CH₂-, -CH₂SO₂NH-, -CH₂N(CH₃)SO₂CH₂CH₂-, -N(SO₂Me)CH₂CH₂-, -CH₂C(O)NH- or - CH₂NHC(O)CH₂-. A C₂-C₄alkenylene chain which may be uninterrupted or interrupted by oxygen is accordingly to be understood as being, for example, -CH=CH-CH₂-, - CH=CH-CH₂CH₂- or -CH=CHCH₂OCH₂-, and a C₂-C₄alkynylene chain which may be uninterrupted or interrupted by oxygen is to be understood as being, for example, -C≡C-, -C≡CCH₂-, -C≡CCH₂O-, -C≡CCH₂OCH₂- or -OC≡CCH₂-.

A three- to ten-membered mono- or bi-cyclic ring system Ra₁ or Ra₂, which may be interrupted once or up to three times selected from oxygen, sulfur, S(O), SO₂, N(Ra₆), carbonyl and C(=NORa₇) and which is bonded to the carbon atom of the substituent A₁ or to the group Q₁ or Q₂ either directly or by way of a C₁-C₄alkylene, C₁-C₄alkenylene or C₂-C₄alkynylene bridge which may be interrupted by oxygen, -N(C₁-C₄alkyl)-, sulfur, sulfinyl and/or sulfonyl, is to be understood as being, for example, 1-methyl-1H-pyrazol-3-yl, 1-ethyl-1H-pyrazol-3-yl, 1-propyl-1H-pyrazol-3-yl, 1H-pyrazol-3-yl, 1,5-dimethyl-1H-pyrazol-3-yl, 4-chloro-1-methyl-1H-pyrazol-3-yl, 1H-pyrazol-1-yl, 3-methyl-1H-pyrazol-1-yl, 3,5-dimethyl-1H-pyrazol-l-yl, 3-isoxazolyl, 5-methyl-3-isoxazolyl, 3-methyl-5-isoxazolyl, 5-isoxazolyl, 1H-pyrrol-2-yl, 1-methyl-1H-pyrrol-2-yl, 1H-pyrrol-1-yl, 1-methyl-1H-pyrrol-3-yl, 2-furanyl, 5-methyl-2-furanyl, 3-furanyl, 5-methyl-2-thienyl, 2-thienyl, 3-thienyl, 1-methyl-1H-imidazol-2-yl, 1H-imidazol-2-yl, 1-methyl-1H-imidazol-4-yl, 1-methyl-1H-imidazol-5-yl, 4-methyl-2-oxazolyl, 5-methyl-2-oxazolyl, 2-oxazolyl, 2-methyl-5-oxazolyl, 2-methyl-4-oxazolyl, 4-methyl-2-thiazolyl, 5-methyl-2-thiazolyl, 2-thiazolyl, 2-methyl-5-thiazolyl, 2-methyl-4-thiazolyl, 3-methyl-4-isothiazolyl, 3-methyl-5-isothiazolyl, 5-methyl-3-isothiazolyl, 1-methyl-1H-1,2,3-triazol-4-yl, 2-methyl-2H-1,2,3-triazol-4-yl, 4-methyl-2H-1,2,3-triazol-2-yl, 1-methyl-1H-1,2,4-triazol-3-yl, 1,5-dimethyl-1H-1,2,4-triazol-3-yl, 3-methyl-1H-1,2,4-triazol-1-yl, 5-methyl-1H-1,2,4-triazol-1-yl, 4,5-dimethyl-4H-1,2,4-triazol-3-yl, 4-methyl-4H-1,2,4-triazol-3-yl, 4H-1,2,4-triazol-4-yl, 5-methyl-1,2,3-oxadiazol-4-yl, 1,2,3-oxadiazol-4-yl, 3-methyl-1,2,4-oxadiazol-5-yl, 5-methyl-1,2,4-oxadiazol-3-yl, 4-methyl-3-furazanyl, 3-furazanyl, 5-methyl-1,2,4-oxadiazol-2-yl, 5-methyl-1,2,3-thiadiazol-4-yl, 1,2,3-thiadiazol-4-yl, 3-methyl-1,2,4-thiadiazol-5-yl, 5-methyl-1,2,4-thiadiazol-3-yl, 4-methyl-1,2,5-thiadiazol-3-yl, 5-methyl-1,3,4-thiadiazol-2-yl, 1-methyl-1H-tetrazol-5-yl, 1H-tetrazol-5-yl, 5-methyl-1H-tetrazol-1-yl, 2-methyl-2H-tetrazol-5-yl, 2-ethyl-2H-tetrazol-5-yl, 5-methyl-2H-tetrazol-2-yl, 2H-tetrazol-2-yl, 2-pyridinyl, 6-methyl-2-pyridinyl, 4-pyridinyl, 3-pyridinyl, 6-methyl-3-pyridazinyl, 5-methyl-3-pyridazinyl, 3-pyridazinyl, 4,6-dimethyl-2-pyrimidinyl, 4-methyl-2-pyrimidinyl, 2-pyrimidinyl, 2-methyl-4-pyrimidinyl, 2-chloro-4-pyrimidinyl, 2,6-dimethyl-4-pyrimidinyl, 4-pyrimidinyl, 2-methyl-5-pyrimidinyl, 6-methyl-2-pyrazinyl, 2-pyrazinyl, 4,6-dimethyl-1,3,5-triazin-2-yl, 4,6-dichloro-1,3,5-triazin-2-yl, 1,3,5-triazin-2-yl, 4-methyl-1,3,5-triazin-2-yl, 3-methyl-1,2,4-triazin-5-yl, 3-methyl-1,2,4-triazin-6-yl, 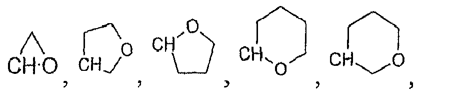 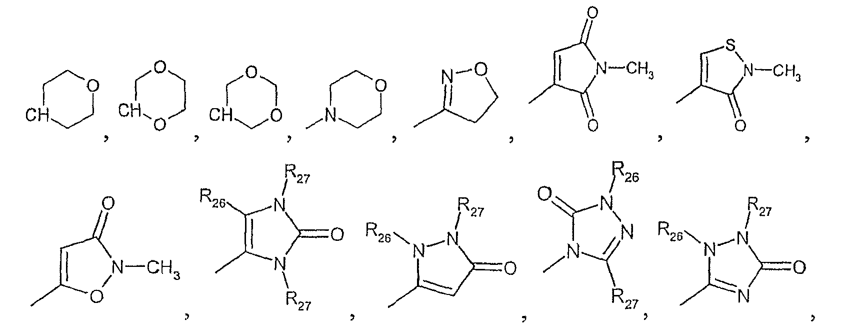 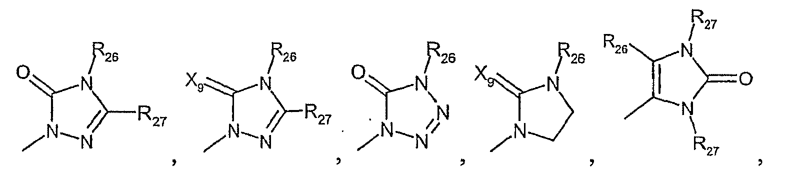 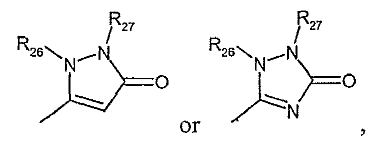 wherein each R₂₆ is methyl, each R₂₇ independently is hydrogen, C₁-C₃alkyl, C₁-C₃alkoxy, C₁-C₃alkylthio or trifluoromethyl, and X₉ is oxygen or sulfur.

A further annellated (fused-on), monocyclic or bicyclic ring system which is formed, for example, by two adjacent substituents Ra₁ and Ra₂ or Ra₁ and Ra₅ and which is uninterrupted or interrupted once or up to three times selected from oxygen, sulfur, S(O), SO₂, -N(Ra₆)- carbonyl and C(=NORa₇) and which may be additionally substituted by one or more substituents is to be understood as being, for example, an annellated, bidentate ring system of formula 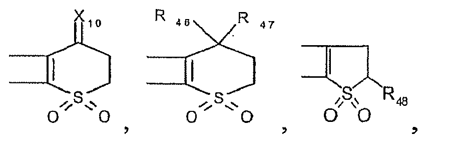 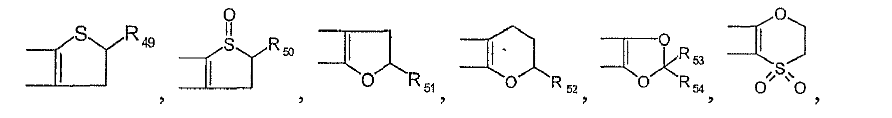 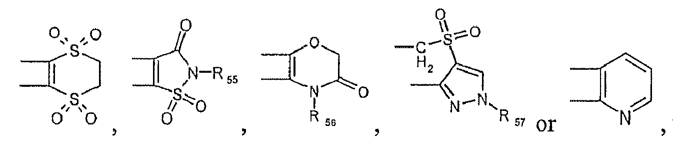 wherein especially R₄₆ is hydrogen, halogen, C₁-C₄alkyl, C₁-C₄haloalkyl, C₁-C₄alkoxy or C₁-C₄alkylthio; R₄₇ is hydrogen, halogen, C₁-C₄alkyl or C₁-C₄alkoxy; and R₅₀, R₅₁, R₅₂; R₅₃, R₅₄, R₅₅, R₅₆, R₅₇, R₅₈ and R₅₉ are hydrogen or C₁-C₄alkyl; and X₁₀ is oxygen or NOR₅₉.

A number of HPPD inhibitors of formula I are described within the art.

In a particular embodiment of the invention the HPPD inhibitor comprises the compound of formula I wherein:
T is T₁;
R₁ and R₂ are hydrogen;
A is C₁-C₂alkylene;
D and E together are C₂-C₃alkylene;
Q is Q₁, wherein
A₁ is methine, CRa₁ or =N-(O)ₚ, but preferably =N-(O)ₚ;
pis 0;
Ra₁ is hydrogen, C₁-C₆alkyl, hydroxy, C₁-C₆alkoxy, C₁-C₆haloalkoxy, C₃-C₆alkenyloxy, C₃-C₆haloalkenyloxy, C₃-C₆alkynyloxy, C₁-C₄alkylcarbonyloxy, C₁-C₄alkylsulfonyloxy, tosyloxy, C₁-C₄alkylthio, C₁-C₄alkylsulfinyl, C₁-C₄alkylsulfonyl, C₁-C₄alkylamino, di-C₁-C₄alkylamino, C₁-C₄alkoxycarbonyl, C₁-C₄haloalkyl, formyl, cyano, halogen, phenyl or phenoxy; it being possible for phenyl in turn to be substituted by C₁-C₃alkyl, C₁-C₃haloalkyl, C₁-C₃alkoxy, C₁-C₃haloalkoxy, halogen, cyano or by nitro; Ra₂ is hydrogen, C₁-C₆alkyl, C₁-C₆haloalkyl, C₂-C₆alkenyl, C₂-C₆haloalkenyl, vinyl substituted by C₁-C₂alkoxycarbonyl or by phenyl, C₂-C₆alkynyl, C₂-C₆haloalkynyl, ethynyl substituted by trimethylsilyl, hydroxy, C₁-C₂alkoxy, C₁-C₂alkoxycarbonyl or by phenyl, C₃-C₆allenyl, C₃-C₆cycloalkyl, halo-substituted C₃-C₆cycloalkyl, C₁-C₆alkoxy, C₃-C₆alkenyloxy, C₃-C₆alkynyloxy, C₁-C₆haloalkoxy, C₃-C₆haloalkenyloxy, cyano-C₁-C₄alkoxy, C₁-C₄alkoxy-C₁-C₄alkoxy, C₁-C₄alkylthio-C₁-C₄alkoxy, C₁-C₄alkylsulfinyl-C₁-C₄alkoxy, C₁-C₄alkylsulfonyl-C₁-C₄alkoxy, C₁-C₄alkoxycarbonyl-C₁-C₄alkoxy, C₁-C₆alkylthio, C₁-C₆allcylsulfinyl, C₁-C₆alkylsulfonyl, C₁-C₆haloalkylthio, C₁-C₆haloallcylsulfinyl, C₁-C₆haloalkylsulfonyl, C₁-C₄alkoxycarbonyl-C₁-C₄alkylthio, C₁-C₄alkoxycarbonyl-C₁-C₄alkylsulfinyl, C₁-C₄alkoxycarbonyl-C₁-C₄alkylsulfonyl, benzyl-S-, benzyl-SO-, benzyl-SO₂-, C₁-C₆alkylamino, di-C₂-C₆alkylamino, C₁-C₆alkylaminosulfonyl, di(C₁-C₆alkylamino)sulfonyl, benzyloxy, benzyl, phenyl, phenoxy, phenylthio, phenylsulfinyl or phenylsulfonyl, it being possible for the phenyl-containing groups in turn to be substituted by C₁-C₃alkyl, C₁-C₃haloalkyl, C₁-C₃alkoxy, C₁-C₃haloalkoxy, halogen, cyano or by nitro, or Ra₂ is OS-C₁-C₄alkyl, OSO-C₁-C₄alkyl, OSO₂-C₁-C₄alkyl, OS-C₁-C₄haloalkyl, OSO-C₁-C₄haloalkyl, OSO₂-C₁-C₄haloalkyl, N(C₁-C₄alkyl)-S-C₁-C₄alkyl, N(C₁-C₄alkyl)-SO-C₁-C₄alkyl, N(C₁-C₄alkyl)-SO₂-C₁-C₄alkyl, cyano, carbamoyl, C₁-C₄alkoxycarbonyl, formyl, halogen, rhodano, amino, hydroxy-C₁-C₄alkyl, C₁-C₄alkoxy-C₁-C₄alkyl, C₁-C₄alkyl-S-C₁-C₄alkyl, C₁-C₄alkyl-SO-C₁-C₄alkyl, C₁-C₄alkyl-SO₂-C₁-C₄alkyl, cyano-C₁-C₄alkyl, C₁-C₆alkylcarbonyloxy-C₁-C₄alkyl, C₁-C₄alkoxycarbonyl-C₁-C₄alkyl, C₁-C₄alkoxycarbonyloxy-C₁-C₄alkyl, C₁-C₄-rhodano-C₁-C₄alkyl, benzoyloxy-C₁-C₄alkyl, C₂-C₆oxiranyl, C₁-C₄alkylamino-C₁-C₄alkyl, di(C₁-C₄alkyl)amino-C₁-C₄alkyl, C₁-C₁₂alkylthiocarbonyl-C₁-C₄alkyl or formyl-C₁-C₄alkyl, or Ra₂ is the group -X₁-X₃ or the group -X₂-X₁-X₃; wherein X₁, X₂ and X₃ are as defined above;
Ra₃ and Ra₄ are hydrogen and Ra₅ is as defined above.

In a still further embodiment the invention the HPPD inhibitor comprises a compound of formula I wherein:
T is T₁;
R₁ and R₂ are hydrogen, A is methylene, D and E together are ethylene, A₁ is =N-(O)ₚ; wherein p is 0;
Q is Q₁, Ra₃ and Ra₄ are hydrogen, Ra₅ is C₁-C₃haloalkyl, especially trifluoromethyl, and Ra₂ is C₁-C₄alkoxy-C₁-C₄alkoxy-C₁-C₄alkyl, especially methoxyethoxymethyl.

HPPD inhibiting compounds are well known in the art and there are numerous tests that can be employed to identify the capacity of a test compound to inhibit HPPD. For example, *in vitro* screening assays as described in the examples of the present application may be use or alternative *in vitro* screening methods can be employed such as the method described in example 11 of WO02/46387 wherein a known HPPD enzyme is selected and a test inhibitor compound is applied.

In a still further embodiment of the invention the HPPD inhibitor is 2-(2-Nitro-4-Trifluoromethylbenzoyl)-1,3-Cyclohexanedione (compound 2). The term compound 2 may be interchanged with formula II). It will be appreciated that 2-(2-Nitro-4-Trifluoromethylbenzoyl)-1,3-Cyclohexanedione (compound 2) may exist in one or more tautomeric forms, one of which is shown in formula (II) (i.e. compound 2) : and which forms are readily inter-convertible by keto-enol tautomerism.

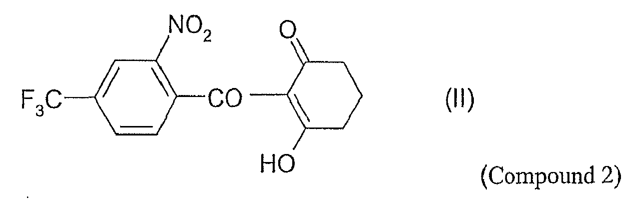

It is to be understood that the invention includes the use of 2-(2-Nitro-4-Trifluoromethylbenzoyl)-1,3-Cyclohexanedione in any of such tautomeric forms or as a mixture thereof.

2-(2-Nitro-4-Trifluoromethylbenzoyl)-1,3-Cyclohexanedione is acidic and readily forms salts with a wide variety of bases.

Particularly suitable salts of 2-(2-Nitro-4-Trifluoromethylbenzoyl)-1,3-Cyclohexanedione suitable for use as active ingredients in pharmaceutical compositions according to the invention include, for example, pharmaceutically acceptable base-addition salts, for example, alkali metal (such as potassium or sodium), alkaline earth metal (such as calcium or magnesium) and ammonium salts, and salts with organic bases giving physiologically acceptable cations (such as salts with methylamine, dimethylamine, trimethylamine, piperidine and morpholine).

2-(2-Nitro-4-Trifluoromethylbenzoyl)-1,3-Cyclohexanedione may be obtained by conventional procedures of organic chemistry already known for the production of structurally analogous materials.

Thus, for example, 2-(2-Nitro-4-Trifluoromethylbenzoyl)-1,3-Cyclohexanedione may be conveniently obtained by reaction of 2-nitro-4-trifluoromethylbenzoyl chloride with cyclohexane-1,3-dione in the presence of acetone cyanhydrin and a suitable base such as triethylamine.

The starting 2-nitro-4-trifluoromethylbenzoyl chloride may itself be obtained from the corresponding benzoic acid, for example by reaction with thionyl chloride or oxalyl chloride as is described in Reagents for Organic Synthesis, (J Wiley and Sons, 1967; editors: Fieser L. F. and Fieser M.; Vol 1, pp. 767-769) and is generally used without special purification.

Similarly, 2-nitro-4-trifluroromethylbenzoic acid may be obtained, for example, as described by Haupstein et al. in J. Amer. Chem. Soc., 1954, 76, 1051, or by one of the general methods well known to the skilled person.

In a still further embodiment of the invention the HPPD inhibitor or precursor is a compound having the structure depicted in Table A below.

**Table A.**

| | Compound Number | Structure: |
|---|---|---|
| | 3.01 | 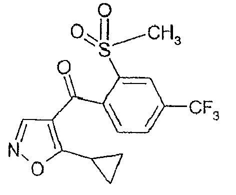 |
| | 3.02 | 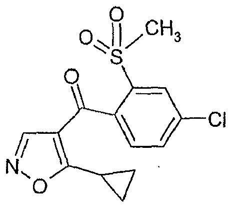 |
| | 3.03 | 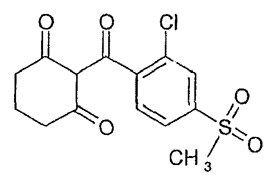 |
| | 3.04 | 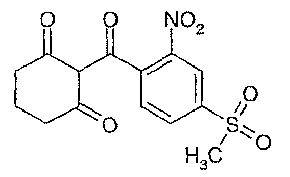 |
| | 3.05 | 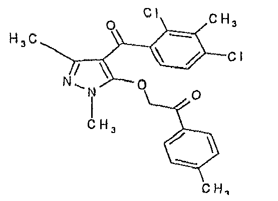 |
| | 3.06 | 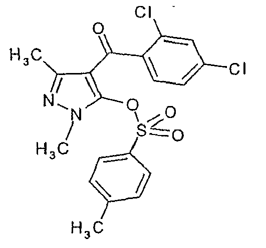 |
| | 3.07 | 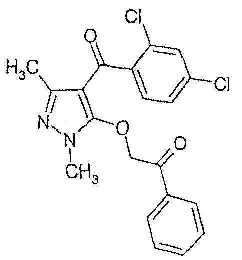 |
| | 3.08 | 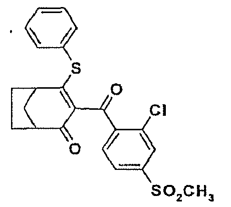 |
| | 3.09 | 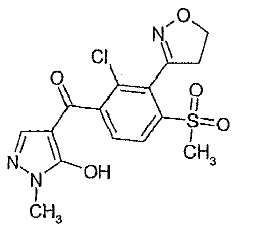 |
| | 3.10 | 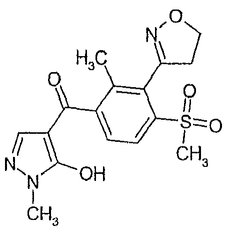 |
| | 3.11 | 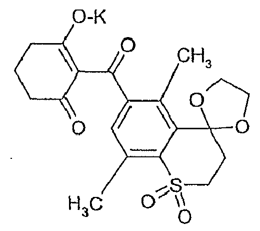 and the free acid thereof |
| | 3.12 | 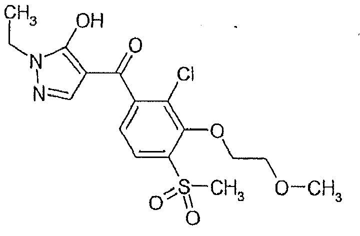 |
| | 3.13 | 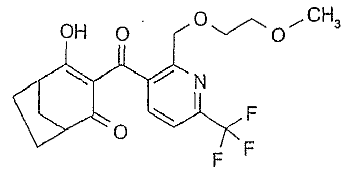 |
| | 3.14 | 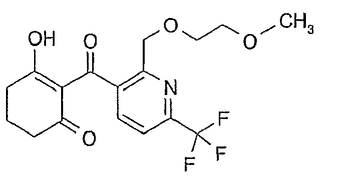 |
| | 3.15 | 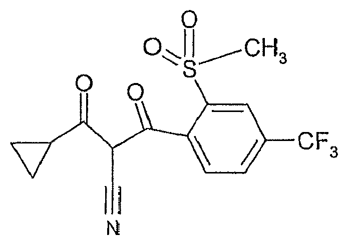 |
| | 3.16 | 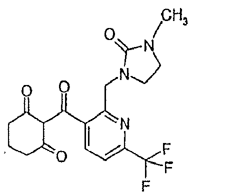 |
| | 3.17 | 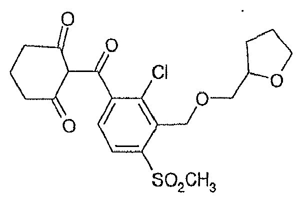 |
| | 3.18 | 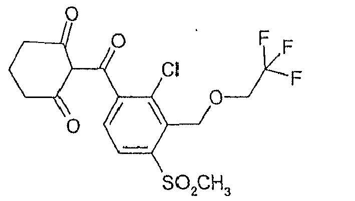 |
| | 3.19 | 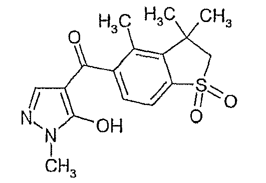 |
| | 3.20 | 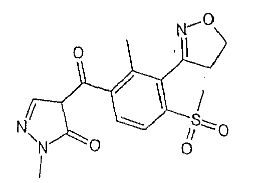 |
| | 3.21 | 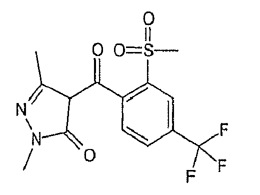 |
| | 3.22 | 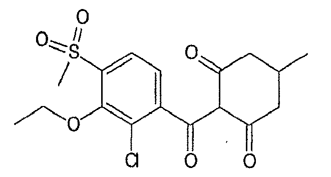 |
| | 3.23 | 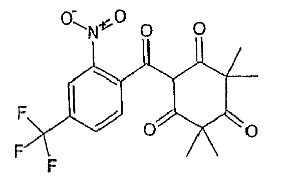 |
| | 3.24 | 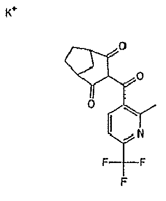 |
| | 3.25 | 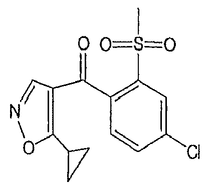 |
| | 3.26 | 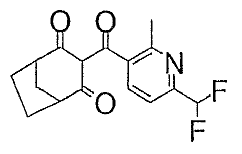 |

In addition to the compounds described herein, it is also possible to use a compound which is a precursor to an HPPD inhibiting compound.

A "precursor" is a compound which itself is not an HPPD inhibitor but is metabolised to produce an HPPD inhibitor for use in accordance with the present invention. For example the compound depicted as compound No. 3.01 in Table A above is a precursor to the compound depicted as compound No. 3.15.

Thus, throughout this specification, "HPPD inhibitor" includes those compounds which are capable of inhibiting HPPD in animals and any precursor compound thereof which is capable of being metabolised in the animal to produce the HPPD inhibiting compound.

It will also be appreciated that alternative steps in the tyrosine catabolism pathway may be inhibited in addition to or as an alternative to the inhibition of HPPD. For example, inhibitors of enzymes/compounds that are "upstream" of HPPD in said pathway such as tyrosine aminotransferase, may be used and/or likewise inhibitors of enzymes/compounds "downstream" of HPPD in said pathway such as homogentisic acid oxidase may also be used.

The present invention still further provides the use as described above wherein said medicament is administered in combination with an anti-inflammatory agent.

The present invention still further provides the use as described above wherein said medicament comprises an anti-inflammatory agent.

The present invention still further provides the use as described above wherein said medicament comprises a first HPPD inhibitor and a further HPPD inhibitor and wherein said first inhibitor is different from said further inhibitor and wherein said inhibitors are present in amount to treat and/or prevent depression and/or treat the withdrawal symptoms in an animal associated with an addictive drug which causes dopamine dependant associative learning disorders in said animal.

In a particular embodiment said first and further HPPD inhibitor is selected from an inhibitor described above.

In a still further embodiment said first and/or second compound comprises a precursor compound.

In a still further aspect of the invention there is provided a kit comprising a pharmaceutically effective amount of a first HPPD inhibitor and a further HPPD inhibitor and wherein said first inhibitor is different from said further inhibitor. In a particular embodiment said first and further HPPD inhibitor is selected from an inhibitor described above and wherein said inhibitors are present in amount to treat and/or prevent depression and/or treat the withdrawal symptoms in an animal associated with an addictive drug which causes dopamine dependant associative learning disorders in said animal.

In a still further embodiment said first inhibitor comprises 2-(2-Nitro-4-Trifluoromethylbenzoyl)-1,3-Cyclohexanedione (compound 2). In a still further embodiment said first inhibitor comprises the compound depicted as 3.22 as described above.

The invention will now be described by way of the following non-limiting examples and Figure of which:

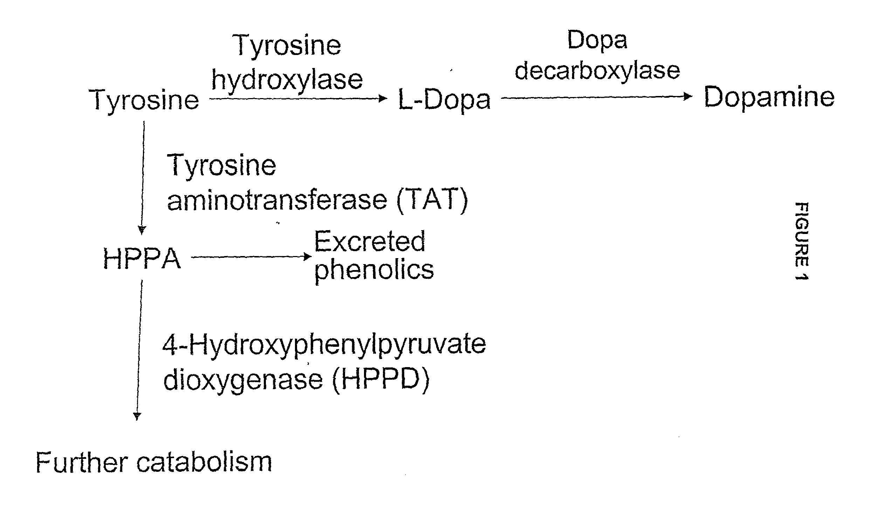

Figure 1 is a representation of part of a pathway indicating the metabolism of tyrosine.

### EXAMPLES

### Example 1

Illustration of increase in levels and turnover of dopamine as a mechanism for treatment/prevention of depression and treatment of the withdrawal symptoms associated with withdrawal from an addictive drug such as cocaine.

10 mice were dosed orally with 0.1 mg/Kg of 2-(2-Nitro-4-Trifluoromethylbenzoyl)-1,3-Cyclohexanedione.
As a control, 10 mice were orally dosed with the dosing vehicle.
After 24 hours, plasma tyrosine levels were identified. Dopamine levels and dopamine turnover were also analysed.

The results yielded mice dosed with 2-(2-Nitro-4-Trifluoromethylbenzoyl)-1,3-Cyclohexanedione had elevated levels of plasma tyrosine.
In addition to this, dopamine was increased by up to 16% and dopamine turnover was increased by up to 27% when compared with the mean of the controls.

### Example 2

10 mice were dosed orally with 0.1 mg/Kg of 2-(2-Nitro-4-Trifluoromethylbenzoyl)-1,3-Cyclohexanedione.
As a control, 9 mice were orally dosed with the dosing vehicle.
After 6 hours, plasma tyrosine levels were identified. Dopamine levels and dopamine turnover were also analysed.
The results yielded mice dosed with 2-(2-Nitro-4-Trifluoromethylbenzoyl)-1,3-Cyclohexanedione had elevated levels of plasma tyrosine.
In addition to this, dopamine was increased by up to 15% and dopamine turnover was increased by up to 15% when compared with the mean of the controls.

### Example 3

10 rats were dosed orally with 2 mg/Kg of 2-(2-Nitro-4-Trifluoromethylbenzoyl)-1,3-Cyclohexanedione.
As a control, 10 rats were orally dosed with the dosing vehicle.
After 24 hours, plasma tyrosine levels were identified. Dopamine levels and dopamine turnover and noradrenaline levels were also analysed.

The results yielded rats dosed with 2-(2-Nitro-4-Trifluoromethylbenzoyl)-1,3-Cyclohexanedione had elevated levels of plasma tyrosine.
In addition to this, dopamine turnover was increased by 25% in the treated compared with the control group.
Furthermore, noradrenaline was increased by up to 76% and 21% in the treated rats cortex and hypothalamus when compared with the mean of the controls.

### Example 4

Analysis of stimulatory effect of HPPD inhibitors as a mechanism for the treatment of withdrawal symptoms associated with an addictive drug such as nicotine.

12 rats were dosed orally with 2 mg/Kg of 2-(2-Nitro-4-Trifluoromethylbenzoyl)-1,3-Cyclohexanedione.
As a control, 12 rats were orally dosed with the dosing vehicle.
After 4 hours, animals were each placed in an activity cage for one hour, containing an automatic activity monitoring system with infrared beams to determine activity and mobility.

Analysis of the activity data indicated that mean rearing counts, centre rearing counts and rearing time were increased by 91%, 63% and 79%, respectively, when compared with the control group. In addition to this, activity counts, active time and mobile time were decreased by approximately 43%, 27% and 49%, respectively, when compared with the control group. The increase in rearing activity indicated that the test substance had a mild stimulatory effect on this behaviour. Increased time spent rearing resulted in the animals spending less time being mobile, which is why indicators of mobility were decreased.

Further expressions of the inventive concept are set out in the following Clauses:
1. The use of at least one compound capable of inhibiting 4-hydroxyphenylpyruvate dioxygenase (HPPD) in an animal in the manufacture of a medicament for use in the treatment and/or prevention of depression.
2. The use according to Clause 1 wherein said medicament comprises a compound selected from the group consisting of: compound 1; 2; and 3.22.
3. The use according to Clause 1 or Clause 2 wherein said medicament comprises the compound depicted as compound 1.
4. The use according to any one of the previous clauses wherein said medicament comprises a selective serotonin reuptake inhibitor (SSRI) and/or a tricyclic antidepressant (tricyclic).
5. The use according to Clause 4 wherein said SSRI is selected from the group consisting of: citalopram, escitalopram, fluoxetine, fluvoxamine, paroxetine, and sertraline or mixtures thereof and said tricyclic is selected from the group consisting of: amitriptyline, clomipramine, dosulepin, dothiepin, doxepin, maprotiline, mianserin, trazodone, trimipramine, amoxapine, imipramine, lofepramine, and nortriptyline or mixtures thereof.
6. A kit comprising a pharmaceutically effective amount of a compound selected from the group consisting of: compound 1; 2; and 3.22 or a pharmaceutically acceptable salt thereof and a pharmaceutically effective amount of a selective serotonin reuptake inhibitor and/or a tricyclic antidepressant and a means for the delivery thereof to an animal.
7. A pharmaceutical composition comprising as an active ingredient a pharmaceutically effective amount of a compound selected from the group consisting of compound 1; 2; and 3.22 or a pharmaceutically acceptable salt thereof and a pharmaceutically effective amount of a selective serotonin reuptake inhibitor and/or a tricyclic antidepressant together with a pharmaceutically acceptable diluent or carrier.
8. A pharmaceutical composition according to Clause 7 wherein said SSRI is selected from the group consisting of: citalopram, escitalopratn, fluoxetine, fluvoxamine, paroxetine, and sertraline or mixtures thereof and said tricyclic is selected from the group consisting of: amitriptyline, clomipramine, dosulepin, dothiepin, doxepin, maprotiline, mianserin, trazodone, trimipramine, amoxapine, imipramine, lofepramine, and nortriptyline or mixtures thereof.
9. The use of at least one compound capable of inhibiting 4-hydroxyphenylpyruvate dioxygenase (HPPD) in an animal in the manufacture of a medicament for use in the treatment of the withdrawal symptoms associated with an addictive drug which causes dopamine dependant associative learning disorders in said animal.
10. The use according to Clause 9 wherein said addictive drug is a drug selected from the group consisting of: cocaine, amphetamine, opiate, nicotine, ethanol, tetrahydrocannabinol and phencyclidine.
11. The use according to Clause 10 wherein said medicament comprises a compound selected from the group depicted as compound 1; 2; and 3.22.
12. The use according to any one of Clauses 9 to 11 wherein the medicament comprises a further compound which is also capable of inhibiting 4-hydroxyphenylpyruvate dioxygenase (HPPD) in an animal.
13. The use according to any one of Clauses 9 to 12 wherein said medicament comprises a nicotine replacement therapy.
14. The use according to any one of Clauses 9 to 13 wherein said medicament comprises bupropion.
15. The use according to any one of Clauses 9 to 14 wherein said medicament is used in conjunction with a nicotine replacement therapy.
16. The use according to any one of Clauses 9 to 15 wherein said medicament is used in conjunction with bupropion.
17. A kit comprising a pharmaceutically effective amount of compound selected from the group consisting of: 1; 3.22; and 2 or a pharmaceutically acceptable salt thereof and a pharmaceutically effective amount of a nicotine replacement t therapy and/or bupropion and a means for the delivery thereof to an animal.
18. A pharmaceutical composition comprising as an active ingredient a pharmaceutically effective amount of a compound selected from the group consisting of: compound 1; 3.22; and 2 or a pharmaceutically acceptable salt thereof and a pharmaceutically effective amount of a nicotine replacement therapy and/or bupropion together with a pharmaceutically acceptable diluent or carrier.
19. A pharmaceutical composition according to Clause 7, 8 and 18 which is in a form suitable for oral or parenteral administration.
20. A pharmaceutical composition according to Clause 19 which is in palatable form suitable for oral administration selected from the group consisting of: tablets; lozenges; hard capsules; aqueous suspensions; oily suspensions; emulsions; dispersible powders; dispersible granules; syrups and elixirs.
21. A pharmaceutical composition according to Clause 19 or 20 which is intended for oral use and is in the form of hard or soft gelatin capsules.
22. A pharmaceutical composition as defined in Clause 19 which is in a form suitable for parenteral administration.
23. A method of treating and/or preventing depression comprising administering to an animal a pharmaceutically effective amount of a compound selected from the group consisting of: compound 1; 2; and 3.22 or a composition according to any one of Clauses 7, 8 and 18 to 22.
24. A method of treating an animal suffering from withdrawal symptoms resulting from addiction to a drug which is responsible for the development of dopamine dependant associative learning disorders in said animal comprising administering to said animal a pharmaceutically effective amount of a compound selected from the group consisting of: compound 1; 3.22; and 2 or a composition according to any one of Clauses 7, 8 and 18 to 22.
25. A method according to Clause 23 or 24 wherein said animal is a human being.

## Claims

1. Use of at least one compound capable of inhibiting 4-hydroxyphenylpyruvate dioxygenase (HPPD) in the manufacture of a medicament for use in the treatment and/or prevention of depression.

2. The use according to Claim 1 wherein said medicament comprises a compound selected from the group consisting of: compound 1; 2; and 3.22; and preferably wherein said medicament comprises the compound depicted as compound 1.

3. The use according to Claim 1 or Claim 2, wherein said medicament comprises a selective serotonin reuptake inhibitor (SSRI) and/or a tricyclic antidepressant (tricyclic); and preferably wherein said SSRI is selected from the group consisting of: citalopram, escitalopram, fluoxetine, fluvoxamine, paroxetine, and sertraline or mixtures thereof and said tricyclic is selected from the group consisting of: amitriptyline, clomipramine, dosulepin, dothiepin, doxepin, maprotiline, mianserin, trazodone, trimipramine, amoxapine, imipramine, lofepramine, and nortriptyline or mixtures thereof.

4. A kit comprising a pharmaceutically effective amount of a compound selected from the group consisting of: compound 1; 2; and 3.22 or a pharmaceutically acceptable salt thereof; a pharmaceutically effective amount of a selective serotonin reuptake inhibitor and/or a tricyclic antidepressant; and a means for the delivery thereof to an animal.

5. A pharmaceutical composition comprising as an active ingredient a pharmaceutically effective amount of a compound selected from the group consisting of compound 1; 2; and 3.22 or a pharmaceutically acceptable salt thereof and a pharmaceutically effective amount of a selective serotonin reuptake inhibitor and/or a tricyclic antidepressant together with a pharmaceutically acceptable diluent or carrier; and preferably wherein said SSRI is selected from the group consisting of: citalopram, escitalopratn, fluoxetine, fluvoxamine, paroxetine, and sertraline or mixtures thereof and said tricyclic is selected from the group consisting of: amitriptyline, clomipramine, dosulepin, dothiepin, doxepin, maprotiline, mianserin, trazodone, trimipramine, amoxapine, imipramine, lofepramine, and nortriptyline or mixtures thereof.

6. Use of at least one compound as defined in Claim 1 or Claim 2 in the manufacture of a medicament for use in the treatment of the withdrawal symptoms associated with an addictive drug which causes dopamine dependant associative learning disorders in said animal.

7. The use according to Claim 6, wherein said addictive drug is a drug selected from the group consisting of: cocaine, amphetamine, opiate, nicotine, ethanol, tetrahydrocannabinol and phencyclidine.

8. The use according to Claim 6 or Claim 7, wherein the medicament comprises a further compound which is also capable of inhibiting 4-hydroxyphenylpyruvate dioxygenase (HPPD).

9. The use according to any of Claims 6 to 8, wherein said medicament comprises a nicotine replacement therapy and/or bupropion.

10. The use according to any of Claims 6 to 9 wherein said medicament is used in conjunction with a nicotine replacement therapy, and/or bupropion.

11. A kit comprising a pharmaceutically effective amount of a compound selected from the group consisting of: 1; 3.22; and 2 or a pharmaceutically acceptable salt thereof and a pharmaceutically effective amount of a nicotine replacement therapy and/or bupropion and a means for the delivery thereof to an animal.

12. A pharmaceutical composition comprising as an active ingredient a pharmaceutically effective amount of a compound selected from the group consisting of: compound 1; 3.22; and 2 or a pharmaceutically acceptable salt thereof and a pharmaceutically effective amount of a nicotine replacement therapy and/or bupropion together with a pharmaceutically acceptable diluent or carrier.

13. A pharmaceutical composition according to Claim 5 or Claim 12 which is in a form suitable for oral or parenteral administration.

14. A pharmaceutical composition according to Claim 13 which is in palatable form suitable for oral administration selected from the group consisting of: tablets; lozenges; hard capsules; aqueous suspensions; oily suspensions; emulsions; dispersible powders; dispersible granules; syrups and elixirs; and preferably which is in the form of hard or soft gelatin capsules.

15. A compound as defined in Claim 1 or Claim 2 or a pharmaceutical composition according to any of Claims 5 and 12 to 14 for use in treating and/or preventing depression in an animal.

16. A compound as defined in Claim 1 or Claim 2 or a pharmaceutical composition according to any of Claims 5 and 12 to 14 for use in treating an animal suffering from withdrawal symptoms resulting from addiction to a drug which is responsible for the development of dopamine dependant associative learning disorders in said animal.
